# EUROPEAN PATENT SPECIFICATION

(11) **EP 2 575 836 B1**
(45) Date of publication and mention of the grant of the patent: **02.11.2016**
(21) Application number: 11729176.5
(22) Date of filing: 25.05.2011
(51) Int. Cl.: A61K 35/74, A23L 33/135

(54) **IMMUNE IMPRINTING NUTRITIONAL COMPOSITION**
IMMUNSTÄRKENDE ERNÄHRUNGSZUSAMMENSETZUNG
COMPOSITION NUTRITIONNELLE D'EMPREINTE IMMUNITAIRE

(30) Priority: 25.05.2010 WO PCT/NL2010/050312
(43) Date of publication of application: 10.04.2013
(73) Proprietor: N.V. Nutricia, 2712 HM Zoetermeer (NL)
(72) Inventor: BEN AMOR, Kaouther, NL-3511 PH Utrecht (NL); KNIPPELS, Leon Matthieu Johannes, NL-3523 VJ Utrecht (NL); NAUTA, Alma Jildou, NL-3972 RD Driebergen (NL); GARSSEN, Johan, NL-3433 DA Nieuwegein (NL)
(74) Representative: Nederlandsch Octrooibureau
(86) International application number: PCT/NL2011/050358
(87) International publication number: WO 2011/149346

(56) References cited:
- WO-A1-2008/153377
- KUITUNEN MIKAEL ET AL: "Probiotics prevent IgE-associated allergy until age 5 years in cesarean-delivered children but not in the total cohort.", THE JOURNAL OF ALLERGY AND CLINICAL IMMUNOLOGY FEB 2009 LNKD- PUBMED:19135235, vol. 123, no. 2, February 2009 (2009-02), pages 335-341, XP002616479, ISSN: 1097-6825 cited in the application
- OHNO HIROSHI ET AL: "Oral administration of Bifidobacterium bifidum G9-1 suppresses total and antigen specific immunoglobulin E production in mice", BIOLOGICAL & PHARMACEUTICAL BULLETIN, vol. 28, no. 8, August 2005 (2005-08), pages 1462-1466, XP002616480, ISSN: 0918-6158

## Description

### FIELD OF THE INVENTION

The invention relates to infant nutrition comprising probiotics which has long term effects on the immune system, in particular on IgE levels and cat allergy, going beyond the time the composition has been administered.

### BACKGROUND OF THE INVENTION

Over the past decades, the prevalence of IgE mediated allergic diseases has risen in western countries. This increase has been hypothesized to result from diminished microbial exposure, leading to an altered composition of the intestinal microbiota. It has been shown that intestinal microbiota composition differs between children with and without atopy.

Several randomized controlled trials have been performed to investigate if intestinal microbiota manipulation with probiotics, living micro-organisms with immunomodulatory effects, reduce the severity of atopic dermatitis (AD). Children with AD have a chance of approximately 40% to develop asthma later in childhood, compared to 5-10% in the general population. Since AD is often the starting point of the so-called atopic march, probiotics may bring the atopic march to a halt in these children.

In Kuitinen et al, 2009, J Allerg Clin Immunol 123: 335-341 administration of of a mix of four probiotic strains and galacto-oligosaccharides in high risk infants did not have an overall effects on IgE-associated diseases, except in the subgroup of C-section born infants regarding food allergy (positive SPT response and/or food-specific IgE > 0.7) and IgE-associated eczema. In Kukkonen et al, 2007 J Allergy Clin Immunol. 2007 Jan;119(1):192-8, at the age of 2 years no effects of early administration of a mix of four probiotic strains and galacto-oligosaccharides were observed on the total of IgE associated diseases, except in the subgroup of IgE infants with positive skin prick test responses or serum antigen-specific IgE levels of greater than 0.7 kU/L. The geometric mean serum total IgE level was 25.7 kU/L) in the probiotic group and 27.2 kU/Lin the placebo group (probiotic/placebo ratio, 0.95; 95% CI, 0.76-1.18).

US 2006/233772 discloses the perinatal use of L GG for preventing or reducing the production of serum IgE antibodies in a subject, the method comprising prenatally administering and/or postnatally administering to the subject a therapeutically effective amount ofL GG.

WO 2008/153391 discloses the use of inactivated *B. breve* and a mix of non-digestible oligosaccharides for amongst others treatment and/or prevention of asthma.

### SUMMARY OF THE INVENTION

The inventors have surprisingly found that children, who had been administered a probiotic composition comprising *B. breve* during infancy, had a statistically significant lower specific IgE against cat allergens when examined during a follow up later in life, than those children who had been administered a placebo during infancy. These results indicate that this specific probiotic composition has a preventive effect on cat allergy.

Furthermore, the change in total serum IgE concentration between one year follow-up and baseline was lower in the probiotic than in the placebo group. This effect was especially prevalent in the subgroup of children that were IgE-negative (i.e. had low serum IgE levels) during infancy.

### DETAILED DESCRIPTION OF THE INVENTION

The invention can be worded as a nutritional composition comprising *Bifidobacterium breve* for use in the prevention of cat allergy in a human subject.

### Bifidobacterium breve

The present composition comprises *Bifidobacterium breve. Bifidobacterium breve* is a Grampositive, anaerobic, branched rod-shaped bacterium. The present *B. breve* preferably has at least 95 % identity of the 16 S rRNA sequence when compared to the type strain of *B. breve* ATCC 15700, more preferably at least 97% identity (Stackebrandt & Goebel, 1994, Int. J. Syst. Bacteriol. 44:846-849). Preferred *B. breve* strains are those isolated from the faeces of healthy human milk-fed infants. Typically, these are commercially available from producers of lactic acid bacteria, but they can also be directly isolated from faeces, identified, characterised and produced. According to a preferred embodiment, the present composition contains at least one *B. breve* selected from the group consisting of *B. breve* Bb-03 (Rhodia/Danisco), *B. breve* M-16V (Morinaga), *B. breve* R0070 (Institute Rosell, Lallemand), B. breve BR03 (Probiotical), B. breve BR92) (Cell Biotech), DSM 20091, LMG 11613, YIT4065, FERM BP-6223 and CNCM 1-2219. Most preferably, the *B. breve* is selected from the group consisting of *B. breve* M-16V and *B. breve* CNCM 1-2219.

The present composition preferably contains 10² to 10¹³ colony forming units (cfu) *B. breve* per gram dry weight of the present composition, preferably 10⁴ to 10¹², more preferably 10⁵ to 10¹⁰, most preferably from 10⁵ to 1x10⁹ cfu *B. breve* per gram dry weight of the present composition. The dose of *B. breve* according to the present invention is preferably administered at a daily dose of 10² to 10¹³, more preferably from 10⁵ to 10¹², most preferably from 10⁸ to 5x10¹⁰ colony forming units (cfu). Preferably the composition comprises 10³ to 10¹³ cfu *B. breve* per 100 ml, more preferably 10⁶ to 10¹¹ cfu *B. breve* per 100 ml, most preferably 10⁷ to 10¹⁰ cfu *B. breve* per 100 ml.

The present composition preferably comprises viable *B. breve.* Alternatively, the present composition preferably comprises non-viable *B. breve* equivalent to the amounts of CFU as described above. The equivalent of cfu can be determined by performing the 5'nuclease assay with the *B. breve* probes and primers as disclosed in WO 2005/039319 in the product (i.e. an infant formula) comprising non-viable *B. breve* and compare this with a calibration curve obtained from a comparable product (for instance a standard infant formula) to which known amounts in cfu of viable, preferably dried, *B. breve* have been added. The dried viable bifidobacteria can be commercially obtained as described above. *B. breve* cells can be made non-viable by methods known in the art, including heat treatment steps (including sterilization, pasteurization, UHT treatment), radiation (UV), treatment with oxygen, treatment with bactericidals such as ethanol, sonication, ultra high pressure application, high pressure homogenization and use of a cell disruptor. Preferably the *B. breve* is heat-killed. The presence of non-viable *B. breve* advantageously provides many product technological benefits, including increased shelf-life, a reduced incidence of bacterial contamination, decreased post-acidification of the product, improved dosage control and improved convenience of reconstitution.

The *B. breve* for use according to the present invention is preferably not genetically modified. Genetic modification is disadvantageous with respect to safety and consumer acceptance. Furthermore, genetic modification is costly and usually negatively affects strain growth properties.

### Non-digestible oligosaccharides

Preferably the present composition comprises non-digestible oligosaccharides with a degree of polymerization (DP) between 2 and 250, more preferably 3 and 60. Non-digestible oligosaccharides further support the preventive effect on asthma later in life and on asthma medication. These effects are synergistic. The term "oligosaccharide" as used in the present invention preferably refers to a saccharide with a degree of polymerization (DP) of 2 to 250, preferably a DP 2 to 100, more preferably 2 to 60. It is understood that in the context of this invention a saccharide with a DP in a certain range may include a mixture of saccharides with different average DP's, for example, if an oligosaccharide with a DP of 2 to 100 is included in the present composition, this may include compositions which contain oligosaccharides with an average DP between 2 and 5, an average DP between 50 and 70 and an average DP between 7 and 60. The term "non-digestible oligosaccharide" as used in the present invention refers to oligosaccharides which are not or only partially digested in the intestine by the action of acids or digestive enzymes present in the human upper digestive tract (small intestine and stomach) but which are fermented by the human intestinal flora. For example, sucrose, lactose, maltose and maltodextrins are considered digestible. For example, galacto-oligosaccharides, fructo-oligosaccharides are considered non-digestible oligosaccharide.

The non-digestible oligosaccharide is preferably selected from the group consisting of fructo-oligosaccharides (such as inulin), galacto-oligosaccharides (such as transgalacto-oligosaccharides or beta-galacto-oligisaccharides), gluco-oligosaccharides (such as gentio-, nigero- and cyclodextrin-oligosaccharides), arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides. Preferably the composition comprises gum acacia on combination with a non-digestible oligosaccharide.

Preferably the present composition comprises fructo-oligosaccharides and/or galacto-oligosaccharides, more preferably galacto-oligosaccharides, most preferably transgalacto-oligosaccharides. In a preferred embodiment the composition comprises a mixture of transgalacto-oligosaccharides and fructo-oligosaccharides. Preferably the present composition comprises galacto-oligosaccharides with a DP of 2-10, preferably with an average DP between 2 and 10, and/or fructo-oligosaccharides with a DP of 2-60, preferably with an average DP between 2 and 60, preferably with an average DP between 10 and 60, preferably with an average DP between 20 and 60. Preferably the composition comprises galacto-oligosaccharides and fructo-oligosaccharides in a weight ratio of 20 to 0.5, more preferably 20 to 1, most preferably from 12 to 2.

The galacto-oligosaccharide is preferably selected from the group consisting of transgalacto-oligosaccharides, lacto-N-tetraose (LNT), lacto-N-neotetraose (neo-LNT), fucosyl-lactose, fucosylated LNT and fucosylated neo-LNT. Transgalacto-oligosaccharides ([galactose]ₙ-glucose; wherein n is an integer between 1 and 60, i.e. 2, 3, 4, 5, 6, ...., 59 ,60; preferably n is selected from 2, 3, 4, 5, 6, 7, 8, 9, or 10) are particuoklarly preferred. Transgalacto-oligosaccharides (TOS) are for example sold under the trademark Vivinal™ (Borculo Domo Ingredients, Netherlands). Preferably the saccharides of the transgalacto-oligosaccharides are β-linked.

The present composition preferably contains fructooligosaccharide. The term "fructo-oligosaccharide" as used herein refers to a non-digestible polysaccharide comprising a chain of at least 2 β-linked fructose units, with a DP of 2 to 250, preferably 7 to 100, more preferably 20 to 60. Preferably inulin is used. Inulin is for example available under the tradename "Raftilin HP^{®}", (Orafti). The average DP of the present fructo-oligosaccharide is preferably at least 7, more preferably at least 10, preferably below 100. The fructo-oligosaccharide used preferably has the (majority of) fructose units linked with a β(2→1) linkage. Other terms for fructooligosaccharides include inulin, fructopolysaccharide, polyfructose, fructans and oligofructose. The present composition preferably comprises fructo-oligosaccharides with a DP of 2 to 200.

Preferably, the composition comprises of 80 mg to 2 g non-digestible oligosaccharides per 100 ml, more preferably 150 mg to 1.50 g, even more preferably 300 mg to 1 g per 100 ml. Based on dry weight, the composition preferably comprises 0.25 wt.% to 20 wt.%, more preferably 0.5 wt.% to 10 wt.%, even more preferably 1.5 wt.% to 7.5 wt.% non-digestible oligosaccharides. A lower amount of non-digestible oligosaccharides will be less effective in decreasing IgE levels and cat allergy later in life, whereas a too high amount will result in side-effects of bloating and abdominal discomfort.

Preferably the composition comprises 10² to 10¹³ cfu *B. breve* per gram and 0.25 wt.% to 20 wt.% non-digestible oligosaccharides based on dry weight, more preferably 10⁵ to 10¹⁰ cfu *B. breve* per gram and 0.5 wt.% to 10 wt.% non-digestible oligosaccharides based on dry weight. Preferably the composition comprises 10³ to 10¹³ cfu *B. breve* and 80 mg to 2 g non-digestible oligosaccharides per 100 ml, more preferably 10⁶ to 10¹¹ cfu *B. breve* and 300 mg to 1 g non-digestible oligosaccharides per 100 ml.
Preferably the nutritional composition comprises i) 1x10⁵ cfu to 1x10¹⁰ cfu *B. breve* per g dry weight, more preferably 1x10⁶ cfu to 1x10¹⁰ cfu; and either ii) 0.5 to 20 wt.% galacto-oligosaccharides based on dry weight, more preferably 0.5 to 10 wt.% galacto-oligosaccharides or iii) 0.05 to 2 % fructo-oligosaccharides based on dry weight, more preferably 0.1 to 1 wt.% fructo-oligosaccharides or both ii) and iii).

### Compositions

The present composition is preferably enterally administered, more preferably orally.

The present composition is preferably a nutritional formula, preferably an infant formula. The present composition can advantageously be applied as a complete nutrition for infants. The present composition preferably comprises lipid, protein, and carbohydrate and is preferably administered in liquid form. The present nutritional composition include dry compositions, e.g. powders, which are accompanied with instructions as to admix said dry compositions, in particular nutritional formula, with a suitable liquid, e.g. water.
oligofructose. The present composition preferably comprises fructo-oligosaccharides with a DP of 2 to 200.

Preferably, the composition comprises of 80 mg to 2 g non-digestible oligosaccharides per 100 ml, more preferably 150 mg to 1.50 g, even more preferably 300 mg to 1 g per 100 ml. Based on dry weight, the composition preferably comprises 0.25 wt.% to 20 wt.%, more preferably 0.5 wt.% to 10 wt.%, even more preferably 1.5 wt.% to 7.5 wt.% non-digestible oligosaccharides. A lower amount of non-digestible oligosaccharides will be less effective in decreasing IgE levels and cat allergy later in life, whereas a too high amount will result in side-effects of bloating and abdominal discomfort.

Preferably the composition comprises 10² to 10¹³ cfu *B. breve* per gram and 0.25 wt.% to 20 wt.% non-digestible oligosaccharides based on dry weight, more preferably 10⁵ to 10¹⁰ cfu *B. breve* per gram and 0.5 wt.% to 10 wt.% non-digestible oligosaccharides based on dry weight. Preferably the composition comprises 10³ to 10¹³ cfu *B. breve* and 80 mg to 2 g non-digestible oligosaccharides per 100 ml, more preferably 10⁶ to 10¹¹ cfu *B. breve* and 300 mg to 1 g non-digestible oligosaccharides per 100 ml.
Preferably the nutritional composition comprises i) 1x10⁵ cfu to 1x10¹⁰ cfu *B. breve* per g dry weight, more preferably 1x10⁶ cfu to 1x10¹⁰ cfu; and either ii) 0.5 to 20 wt.% galacto-oligosaccharides based on dry weight, more preferably 0.5 to 10 wt.% galacto-oligosaccharides or iii) 0.05 to 2 wt.% fructo-oligosaccharides based on dry weight, more preferably 0.1 to 1 wt.% fructo-oligosaccharides or both ii) and iii).

### Compositions

The present composition is preferably enterally administered, more preferably orally.

The present composition is preferably a nutritional formula, preferably an infant formula. The present composition can advantageously be applied as a complete nutrition for infants. The present composition preferably comprises lipid, protein, and carbohydrate and is preferably administered in liquid form. The present invention includes dry compositions, e.g. powders,

The infant and/or toddler nutrition for use according to the present invention has been found to be particularly useful as a nutrition for prematurely born babies, maturely born babies (vaginally as well as caesarean section delivered infants), infants which are in the adaptation period to solid food, infants and/or toddlers with an increased risk for or suffering from allergic eczema, from allergy, and/or infants and/or toddlers with an increased risk for infections, such as infants and/or toddlers attending day care centres, or suffering from infections. The invention is particularly advantageous for vaginally born infants. The invention is particularly advantageous for caesarean section delivered infants since these infants have an impaired microbial colonisation of the large intestine and an increased risk on development of IgE associated disorders later in life. The invention is particularly advantageous for vaginally born infants. The invention is particularly advantageous for infants suffering from allergic eczema (also referred to as atopic dermatitis, atopic eczema or allergic dermatitis) since these infants have an increased risk on development of IgE related disorders later in life.

Hence the present nutritional composition is for providing nutrition to a human infant and/or toddler by administering to the infant and/or toddler the present composition. Preferably the infant and/or toddler has an age between 0 and 36 month, more preferably between 0 and 18 month, even more preferably between 0 and 12 months, most preferably between) and 6 months. Preferably the effect on cat allergy and/or on IgE levels is when the human subject has reached an age above 12 months, preferably above 16 month, more preferably above 24 months, more preferably above 36 months, more preferably above 5 years.

Preferably the composition of the present invention is administered for a period of at least 4 weeks, more preferably at least 8 weeks, most preferably at least 12 weeks. A shorter period of administration will result in less effects later in life.

Particularly the present invention provides a composition as described herein above accompanied with indications (e.g. written material) comprising statement that the administration of the composition (e.g. to the infant) prevents cat allergy and/or reduces serum IgE levels later in life.

### EXAMPLES

### Example 1

Ninety full-term infants, aged 0 to 7 months, fulfilling Hanifin and Rajka criteria for atopic dermatitis, were recruited. Inclusion criteria included a SCORing Atopic Dermatitis (SCORAD) score > 15, exclusively formula fed at time of enrolment, no other major medical problems and no use of probiotics or immunomodulatory medication during the 4 weeks before enrolment. Written informed consent was obtained from both parents of all participants.

Participants were randomized, to receive as test composition an extensively hydrolyzed whey based formula (Nutrilon Pepti®, Nutricia, Zoetermeer, the Netherlands) with additional synbiotics or as placebo the same formula without synbiotics for a period of 12 weeks. The investigators, participant's own physicians and parents were all blind to the treatment groups. One year after start of the intervention period participants returned for a follow-up visit, performed by the same investigator, who was still blinded to the treatment groups. During this visit parents were asked about respiratory symptoms (cough, shortness of breath, noisy/rattly breathing, wheezing) and medication use of their child, using a validated questionnaire. Synbiotics consisted of *Bifidobacterium breve* M16-V, 1.3 x 10⁹ cfu/100 ml and a mixture of 90% galacto-oligosaccharides (source Vivinal GOS, Borculo) and 10% fructo-oligosaccharides (source raftilin HP, Orafti), 0.8 g/100 ml. Children with an age below 6 month received starter formula. Children with an age of or above 6 month received follow on formula. Formula was given on demand.

The primary outcome measure of this randomized controlled trial, were change in severity of atopic dermatitis after 12 weeks of intervention. Respiratory outcome measures at follow-up were: 1) prevalence of respiratory symptoms predictive of asthma: frequent wheezing, defined as ≥3 episodes after the intervention period, and wheezing apart from colds, 2) current use of asthma medication (beta-2 agonists, anticholinergics, inhaled corticosteroids), 3) levels of IgE and presence of specific IgE (≥ 0.35 kU/L) against aeroallergens.

At baseline and one-year follow-up total and specific serum IgE against house dust mite (dl), cat (e1) and dog (e2) were determined using the CAP FEIA system (Phadia, Uppsala, Sweden). Specific IgE was considered elevated if ≥ 0.35 kU/L. A subgroup of patients with IgE-negative AD was defined as patients with AD without elevated total and/or specific IgE levels at baseline (total IgE was considered elevated if ≥ 5 kU/L in infants aged < 3 months and ≥ 15 kU/L in infants aged > 3 months, reference values of the Academic Medical Center, Amsterdam).

Parametric data were analyzed with unpaired t-tests. Non-parametric data were analyzed with the Mann-Whitney U test. Binary data were analyzed using the χ²-test, or Fisher's exact test when appropriate, and results are represented as absolute risk reduction (ARR) with 95% confidence intervals (CI). SPSS software (15.0) was used for all analyses.

Ninety infants were randomized in the original study, the intention-to-treat consisted of 85 infants, of which 75 (88%) completed the one-year follow-up evaluation. Baseline characteristics of the children (gender, age, SCORAD index, Breast fed duration, parental asthma, parental smoking, pets, day care, older siblings, probiotics use after intervention period, use of asthma medication, cough, wheezing and noisy/rattling breathing were not statistically different between the two groups. Mean age at follow-up was 17.5 months (SD 1.6) in the test group and 17.2 (SD 1.8) in the placebo group.

Frequent wheezing (≥ 3 episodes after the intervention period), wheezing apart from colds and wheezing and/or noisy/rattly breathing apart from colds were significantly less prevalent in the test group than in the placebo group (ARR of wheezing without colds was significant, however the P value of the χ²-test was 0.056). Significantly fewer children in the test group than in the placebo group used asthma medication at time of follow-up. There were also significantly less new users of asthma medication (children that were using asthma medication at follow-up, but not at baseline) in the test group than in the placebo group.

Median total serum IgE concentrations at baseline and one year follow-up are shown in Table 1. At baseline total IgE was similar in the test group and the placebo group. At follow-up total IgE was lower in the test group than in the placebo group. In the subgroup of children that were IgE-negative at baseline (n=25), total IgE at follow-up was significantly lower in the test group than in the placebo group. This effect was already observed, albeit to a lesser extent, at baseline.

The change in total serum IgE concentration between one year follow-up and baseline (ΔIgE) in the test group and the placebo group is also represented in table 1. In the total study population and the IgE-positive subgroup there was a tendency for a decreased ΔIgE between the test group and the placebo group. In the IgE-negative subgroup ΔIgE was statistically significantly lower in the test group than in the placebo group.

The percentage of children with elevated specific IgE against cat, dog or house dust mite at baseline and one year follow-up is represented in Table 2. At follow-up the percentage of children with positive IgE against cat was significantly lower in the test group than in the placebo group (6.9% vs. 30.3%, *P* = 0.03). The percentage of children with positive IgE against house dust mite (HDM) was also lower in the test group, but this difference was not statistically significant (10.3% vs. 15.2%, *P* = 0.71). No significant difference was observed in the percentage of children with positive IgE against dog allergen.

In conclusion, it was demonstrated that infants that received *B. breve* for a period of 3 months, had a lower prevalence of cat allergy and a reduced increase in IgE, especially in infants being IgE negative at baseline, at one-year follow-up than those who received placebo.

**Table 1. Total IgE levels and change in total IgE concentration**

| | **Test composition** | **Placebo** | ***P* value*** |
|---|---|---|---|
| Total IgE (kU/L), mean ± sem (n) at baseline | 37.73 ± 8.98 (40) | 54.85 ± 17.44 (40) | - |
| Total IgE (kU/L), mean sem (n) at week 12 | 64.83 ± 14.66 (37) | 83.50 ± 20.44 (39) | 0.712* |
| Total IgE (kU/L), mean ± sem (n) at follow up | 82.63 ± 27.06 (29) | 182.71 ± 62.51 (34) | 0.172* |
| IgE-positive, mean ±sem (n) at follow up | 138.85 ± 48.05 (15) | 302.66 ± 111.22 (18) | 0.343* |
| IgE-negative mean ± sem (n) at follow up | 13.28 ± 5.61 (11) | 42.19 ±13.16 (14) | 0.007* |
| Delta IgE (kU/L) median (range) (n) follow-up-baseline Total study population | 5.0 (-14.5-437) (26) | 10.6 (-118-1521) (32) | 0.772** |
| Delta IgE (kU/L) median (range) (n) follow-up-baseline IgE-positive subgroup | 26.7 (-14.5-437) (15) | 13.1 (-118-1521) (18) | 0.704** |
| Delta IgE (kU/L) median (range) (n) follow-up-baseline IgE-negative subgroup | 3.0 (-0.75- 60.9) (11) | 10.6 (-6.84-155) (14) | 0.04 |

| | | | |
|---|---|---|---|
| * ANCOVA analysis with baseline Box-Cox transformation ** Wilcoxon-Mann-Whitney test | | | |

**Table 2. Proportion of positive infants for total and specific IgE (≥ 0.35 kU/L) at baseline, week 12, and follow up in the test group and place (control) group (%).**

| | **Test composition** | **Placebo** | ***P* value*** |
|---|---|---|---|
| Positive total IgE at baseline | 45.0 | 55.0 | 0.503 |
| Positive total IgE at week 12 | 51.4 | 66.7 | 0.243 |
| Positive total IgE at follow up | 44.8 | 55.9 | 0.453 |
| Positive IgE HDM baseline | 0.0 | 2.4 | 1.000 |
| Positive IgE HDM at week 12 | 0.0 | 2.9 | 1.000 |
| Positive IgE HDM at follow up | 10.3 | 15.2 | 0.713 |
| Positive IgE Cat baseline | 9.8 | 12.2 | 1.000 |
| Positive IgE Cat at week 12 | 6.9 | 20.6 | 0.192 |
| Positive IgE Cat at follow up | 6.9 | 30.3 | 0.026 |
| Positive IgE Dog baseline | 5.0 | 3.7 | 1.000 |
| Positive IgE Dog at follow up | 15.0 | 14.8 | 1.000 |

| | | | |
|---|---|---|---|
| * Fisher Exact test | | | |

## Claims

1. A nutritional composition comprising *Bifidobacterium breve* for use in the prevention of cat allergy in a human subject.

2. The nutritional composition for use according to claim 1 wherein the prevention takes place when said human subject has reached an age above 12 months, more preferably above 16 months.

3. The nutritional composition for use according to any one of the preceding claims wherein the nutritional composition is administered to a human subject with an age between 0 and 12 months, more preferably between 0 and 6 months.

4. The nutritional composition for use according to any one of the preceding claims wherein the nutritional composition is administered for a period of at least 4 weeks, more preferably at least 8 weeks.

5. The nutritional composition for use according to any one of the preceding claims wherein said human subject to which the nutritional composition is administered suffers from dermatitis and/or eczema.

6. The nutritional composition for use according to any one of the preceding claims wherein the composition comprises at least 1x10⁵ cfu *B. breve* per g dry weight.

7. The nutritional composition for use according to any one of the preceding claims wherein the *B. breve* is *B. breve* M16-V.

8. The nutritional composition for use according to any one of the preceding claims wherein the nutritional composition further comprises non-digestible oligosaccharides selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides.

9. The nutritional composition for use according to claim 8 wherein the nutritional composition comprises galacto-oligosaccharides and/or fructo-oligosaccharides.

10. The nutritional composition for use according to claim 8 or 9 wherein the nutritional composition comprises more than 0.25 wt.% of non-digestible oligosaccharides based on dry weight.

11. The nutritional composition for use according to any one of the preceding claims wherein the nutritional composition comprises
i. 1x10⁵ cfu to 1x10¹⁰ cfu *B. breve* per g dry weight, and either
ii. 0.5 to 20 wt.% galacto-oligosaccharides based on dry weight or
iii. 0.05 to 2 wt.% fructo-oligosaccharides based on dry weight or both ii. and iii.

12. The nutritional composition for use according to any one of the preceding claims wherein the nutritional composition comprises lipid that provides 5 to 50% of the total calories, protein that provides 5 to 50% of the total calories, and carbohydrate that provides 15 to 90% of the total calories.

13. The nutritional composition for use according to any one of the preceding claims wherein the amount of nutritional composition administered per day is at least 50 ml.

## Patentansprüche

1. Ernährungszusammensetzung, die *Bifidobacterium breve* umfasst, für die Verwendung in der Vorbeugung einer Katzenallergie in einem Menschen.

2. Ernährungszusammensetzung für die Verwendung nach Anspruch 1, wobei die Vorbeugung stattfindet, wenn der Mensch ein Alter von mehr als 12 Monaten, bevorzugter mehr als 16 Monaten, erreicht hat.

3. Ernährungszusammensetzung für die Verwendung nach einem der vorhergehenden Ansprüche, wobei die Ernährungszusammensetzung an einem Menschen in einem Alter von zwischen 0 und 12 Monaten, bevorzugter zwischen 0 und 6 Monaten, verabreicht wird.

4. Ernährungszusammensetzung für die Verwendung nach einem der vorhergehenden Ansprüche, wobei die Ernährungszusammensetzung für einen Zeitraum von wenigstens 4 Wochen, bevorzugter wenigstens 8 Wochen, verabreicht wird.

5. Ernährungszusammensetzung für die Verwendung nach einem der vorhergehenden Ansprüche, wobei der Mensch, dem die Ernährungszusammensetzung verabreicht wird, an einer Dermatitis und/oder einem Ekzem leidet.

6. Ernährungszusammensetzung für die Verwendung nach einem der vorhergehenden Ansprüche, wobei die Zusammensetzung wenigstens 1x10⁵ KBE B. *breve* pro g Trockengewicht umfasst.

7. Ernährungszusammensetzung für die Verwendung nach einem der vorhergehenden Ansprüche, wobei B. *breve B. breve* M16-V ist.

8. Ernährungszusammensetzung für die Verwendung nach einem der vorhergehenden Ansprüche, wobei die Ernährungszusammensetzung ferner nicht verdaubare Oligosaccharide umfasst, die ausgewählt sind aus der Gruppe bestehend aus Fructo-Oligosacchariden, Galacto-Oligosacchariden, Gluco-Oligosacchariden, Arabino-Oligosacchariden, Mannan-Oligosacchariden, Xylo-Oligosacchariden, Fuco-Oligosacchariden, Arabinogalacto-Oligosacchariden, Glucomanno-Oligosacchariden, Galactomanno-Oligosacchariden, Sialinsäure umfassend Oligosaccharide und Uronsäure-Oligosaccharide.

9. Ernährungszusammensetzung für die Verwendung nach Anspruch 8, wobei die Ernährungszusammensetzung Galacto-Oligosaccharide und/oder Fructo-Oligosaccharide umfasst.

10. Ernährungszusammensetzung für die Verwendung nach Anspruch 8 oder 9, wobei die Ernährungszusammensetzung auf der Grundlage des Trockengewichts mehr als 0,25 Gew.-% unverdauliche Oligosaccharide umfasst.

11. Ernährungszusammensetzung für die Verwendung nach einem der vorhergehenden Ansprüche, wobei die Ernährungszusammensetzung umfasst
i. 1x10⁵ KBE bis 1x10¹⁰ KBE *B*. *breve* pro g Trockengewicht, und entweder
ii. 0,5 bis 20 Gew.-% Galacto-Oligosaccharide, basierend auf dem Trockengewicht, oder
iii. 0,05 bis 2% Gew.-% Fructo-Oligosaccharide, basierend auf dem Trockengewicht,
oder sowohl ii. als auch iii.

12. Ernährungszusammensetzung für die Verwendung nach einem der vorhergehenden Ansprüche, wobei die Ernährungszusammensetzung Lipid, das 5 bis 50% der Gesamtkalorien bereitstellt, Protein, das 5 bis 50% der Gesamtkalorien bereitstellt, und Kohlenhydrat, das 15 bis 90% der Gesamtkalorien bereitstellt, umfasst.

13. Ernährungszusammensetzung für die Verwendung nach einem der vorhergehenden Ansprüche, wobei die Menge der pro Tag verabreichten Ernährungszusammensetzung wenigstens 50 ml ist.

## Revendications

1. Une composition nutritionnelle comprenant *Bifidobacterium breve* pour une utilisation pour la prévention de l'allergie au chat chez un sujet humain.

2. La composition nutritionnelle pour une utilisation selon la revendication 1, dans laquelle la prévention se produit quand ledit sujet humain a atteint un âge supérieur à 12 mois, de manière davantage préférée de plus de 16 mois.

3. La composition nutritionnelle pour une utilisation selon l'une quelconque des revendications précédentes, dans laquelle la composition nutritionnelle est administrée à un sujet humain d'un âge compris entre 0 et 12 mois, plus préférablement entre 0 et 6 mois.

4. La composition nutritionnelle pour une utilisation selon l'une quelconque des revendications précédentes, dans laquelle la composition nutritionnelle est administrée pendant une période d'au moins 4 semaines, de préférence encore d'au moins 8 semaines.

5. La composition nutritionnelle pour une utilisation selon l'une quelconque des revendications précédentes, dans lequel ledit sujet humain auquel la composition nutritionnelle est administrée souffre de dermatite et/ou d'eczéma.

6. La composition nutritionnelle pour une utilisation selon l'une quelconque des revendications précédentes, dans laquelle la composition comprend au moins 1 x 10⁵ cfu de *B. breve* par g de poids à sec.

7. La composition nutritionnelle pour une utilisation selon l'une quelconque des revendications précédentes, dans lequel le *B. breve* est *B. breve* M16-V.

8. La composition nutritionnelle pour une utilisation selon l'une quelconque des revendications précédentes, dans laquelle la composition nutritionnelle comprend en outre des oligosaccharides non digestibles sélectionnées dans le groupe constitué des fructo-oligosaccharides, des galacto-oligosaccharides, des gluco-oligosaccharides, des arabino-oligosaccharides, des mannan-oligo-saccharides, des xylo-oligosaccharides, des fuco-oligosaccharides, des arabino-galacto-oligosaccharides, des glucomanno-oligosaccharides, des galactomanno-oligosaccharides, de l'acide sialique comprenant des oligosaccharides et des oligosaccharides d'acide uronique.

9. La composition nutritionnelle pour une utilisation selon la revendication 8, dans laquelle la composition nutritionnelle comprend des galacto-oligosaccharides et/ou des fructo-oligosaccharides.

10. Utilisation selon la revendication 8 ou 9, dans laquelle la composition nutritionnelle comprend en poids plus de 0,25% d'oligosaccharides non digestibles par rapport au poids à sec.

11. La composition nutritionnelle pour une utilisation selon l'une quelconque des revendications précédentes, dans lequel la composition nutritionnelle comprend:
i. 1 x 10⁵ cfu à 1 x 10¹⁰ cfu de *B. breve* par g de poids à sec, et soit
ii. 0,5 à 20% en poids de galacto-oligosaccharides sur la base du poids à sec, ou
iii. 0,05 à 2% en poids de fructo-oligosaccharides par rapport au poids à sec ou les deux, ii. et iii.

12. La composition nutritionnelle pour une utilisation selon l'une quelconque des revendications précédentes, dans laquelle la composition nutritionnelle comprend des lipides qui fournissent 5 à 50% des calories totales, des protéines qui fournissent 5 à 50% des calories totales, et des glucides qui fournissent 15 à 90% des calories totales.

13. La composition nutritionnelle pour une utilisation selon l'une quelconque des revendications précédentes, dans lequel la quantité de composition nutritionnelle administrée par jour est d'au moins 50 ml.
